# EUROPEAN PATENT APPLICATION

(11) **EP 4 534 539 A1**
(43) Date of publication of application: **09.04.2025**
(21) Application number: 23811077.9
(22) Date of filing: 24.05.2023
(51) Int. Cl.: C07D 491/22, A61P 35/00, A61K 31/4745

(54) **DIDEUTERATED CAMPTOTHECIN DERIVATIVE AND PREPARATION METHOD THEREFOR**

(30) Priority: 24.05.2022 CN 202210568947
(71) Applicant: Shanghai Qilu Pharmaceutical Research and Development Centre Ltd., Shanghai 201203 (CN)
(72) Inventor: LV, Wei, Shanghai 200120 (CN); JIN, Jiyu, Shanghai 200120 (CN); JIN, Chen, Shanghai 200120 (CN)
(74) Representative: Dantz, Dirk
(86) International application number: PCT/CN2023/095927
(87) International publication number: WO 2023/227006

(57) **Abstract**

The present invention relates to the technical field of drug synthesis, and provides a dideuterated camptothecin derivative as shown in general formula Q and a preparation method therefor and *in vitro* biological activity and use thereof. The compound is a topoisomerase I inhibitor, and can be used for preparing a drug for treating various diseases in the field related to anti-tumor.

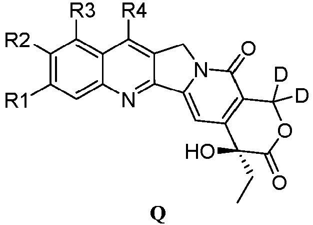

## Description

### TECHNICAL FIELD

The present application relates to the technical field of drug synthesis, and to a dideuterated camptothecin derivative as shown in general formula Q, a preparation method therefor, *in vitro* biological activity, and use thereof. More specifically, the present application relates to a dideuterated camptothecin derivative Q having the following structure, the preparation method therefor, and the use thereof in the manufacture of a medicament and in related fields.

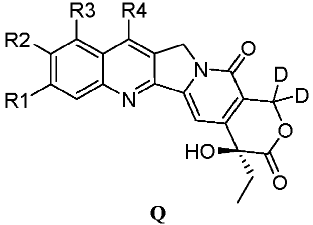

### BACKGROUND

Camptothecin (CPT) is a classic antitumor drug, which is one of the extensively studied natural antitumor drugs besides paclitaxel. The mechanism of CPT is to inhibit topoisomerase I (TOP I) from forming a ternary complex with DNA, thereby rapidly inducing apoptosis of tumor cell. Camptothecin compounds are the only clinically used TOP I inhibitors, and have good therapeutic effect against slow-growing solid tumors in clinic. Three camptothecin compounds have been approved for marketing for the treatment of tumor. Irinotecan, a first-line treatment medicament for colorectal cancer, was first approved by the FDA in 1994; Topotecan was first approved by the FDA in 1996 for the treatment of ovarian cancer; and Belotecan was approved in South Korea in 2005 for the treatment of small cell lung cancer.

There are several problems regarding conventional camptothecin antitumor drugs: 1) poor druggability, camptothecin shows poor lipid solubility and water solubility due to its special structure, and thus requires modification to improve water solubility; 2) camptothecin compounds have certain toxicity, thus high water solubility after the modification may also lead to an instant increase in blood drug level and thereby inducing toxic side effects; and 3) in the modification of camptothecin prodrug, release efficiency and stability need to be considered, which are always a pair of contradictions in rational prodrug design.

With the development of drug delivery system and ADC technology, a series of candidate highly toxic compounds of camptothecins, which have poor druggability and severe side effects, have been in use again. ADC technology can be used to effectively overcome the side effects caused by poor water solubility, insufficient tissue distribution and the like of camptothecin drugs. The powerful targeting ability of antibodies enables ADC molecules as a whole to concentrate in the target tissue, and the toxicity of camptothecin compounds becomes a favorable factor for therapeutic effect instead.

SN-38 is an active metabolite of the antitumor drug Irinotecan on the market, of which the antitumor activity is three orders of magnitude stronger than that of Irinotecan. IMMU-132 is an ADC drug with SN-38 as payload. IMMU-132 was approved for marketing by the FDA in 2020 for the treatment of metastatic triple-negative breast cancer. DS-8201a, another camptothecin ADC drug, was approved for marketing by the FDA in 2019 for metastatic breast cancer that has received at least two anti-HER2 therapies. The GGFG tetrapeptide activated by cathepsin B is used as linker, and a small segment of self-cleavage structure is introduced. The released drug is Dxd, a derivative of Exatecan, which currently shows antitumor effects in HER2-expressing metastatic colorectal cancer.

Deuterated drugs go beyond pure and simple improvements in drug pharmacokinetic parameters, and may offer opportunities when facing problems with metabolically mediated toxicity, drug interactions, and low bioactivity. The more widespread use of deuterium offers the opportunity to reduce the degree of epimerization and reduce the dose of co-administered enhancers.

### SUMMARY

An object of the present application is to provide a dideuterated camptothecin derivative as shown in general formula Q, a pharmaceutical acceptable salt thereof, a stereoisomer thereof, or a pharmaceutical acceptable salt of the stereoisomer thereof. These compounds have inhibitory activity against topoisomerase I.

Another object of the present application is to provide a preparation method of the dideuterated camptothecin derivative as shown in general formula Q.

An additional object of the present application is to provide use of the dideuterated camptothecin derivative in the manufacture of a medicament and in related fields.

The objects of the present application are accomplished as follows.

The present application provides a dideuterated camptothecin derivative as shown in general formula Q, a stereoisomer thereof, a pharmaceutically acceptable salt thereof, or a prodrug thereof, 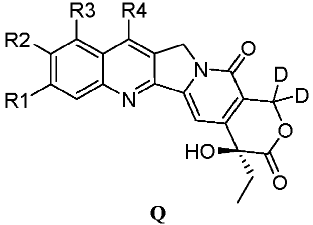 wherein:
R₁ is -H, -CH₃, -(CH₂)ₙCH₃, -OCH₃, -O(CH₂)ₙCH₃, -F, -Cl, -Br, or -I, and n represents an integer from 1 to 3;
R₂ is -H, -OH, -NH₂, -F, -Cl, -Br, -I, -CH₃, -OCH₃, -(CH₂)ₙCH₃, -(CH₂)ₙSi(CH₃)₃, -(CH₂)ₙNH(CH₃)₂, -O(CH₂)ₙCH₃, -O(CH₂)ₙNH₂, -NH(CH₂)ₙNH₂, -(OCH₂CH₂)ₙNH₂, or 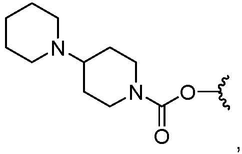 wherein, n represents an integer from 1 to 3; or
R₁ and R₂ are connected to each other to form -(CH₂)ₚ-, -X(CH₂)₍ₚ₋₁₎-, -(CH₂)₍ₚ₋₁₎X-, -X(CH₂)_{(q-1)}X-, or -(CH₂)_{(q-1)}X(CH₂)_{(q-1)}-, p and q each independently represents an integer from 2 to 4, and X represents O, NH, or S;
R₃ is -H, -CH₃, -(CH₂)ₙCH₃, -OCH₃, -O(CH₂)ₙCH₃, -NHCH₃, -NH(CH₂)ₙCH₃, -(CH₂)ₙSi(CH₃)₃, -(CH₂)ₙN(CH₃)₂, -(CH₂)ₙCH=CH₂, -NO₂, -F, -Cl, -Br, or -I, and n represents an integer from 1 to 3;
R₄ is -H, -CH₃, -OCH₃, -(CH₂)ₙCH₃, -Si(CH₃)₂C(CH₃)₃, -(CH₂)ₙSi(CH₃)₃, -(CH₂)ₙNHCH(CH₃)₂, -CH₂NH(CH₂)ₙCH₃, -CH₂O(CH₂)ₙCH₃, -F, -Cl, -Br, -I, 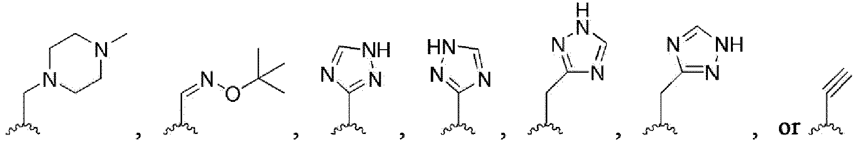 , wherein, n represents an integer from 1 to 3; or
R₃ and R₄ are connected to each other to form:

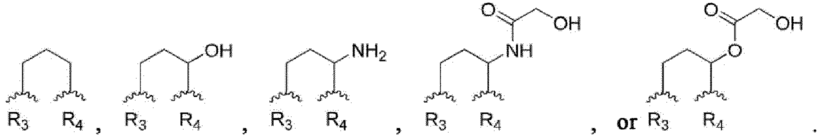

According to an embodiment of the present application, in the general formula Q, 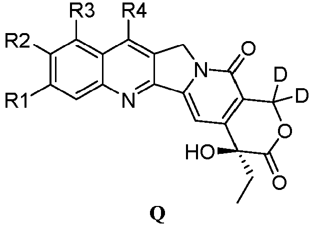
R₁ is -H, -CH₃, -(CH₂)ₙCH₃, -OCH₃, -O(CH₂)ₙCH₃, -F, -Cl, -Br, or -I, and n represents an integer from 1 to 3;
R₂ is -H, -OH, -NH₂, -F, -Cl, -Br, -I, -CH₃, -OCH₃, -(CH₂)ₙCH₃, -(CH₂)ₙSi(CH₃)₃, -(CH₂)ₙNH(CH₃)₂, -O(CH₂)ₙCH₃, -O(CH₂)ₙNH₂, -NH(CH₂)ₙNH₂, -(OCH₂CH₂)ₙNH₂, or 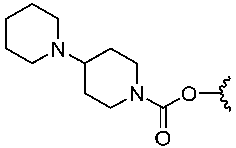 , wherein, n represents an integer from 1 to 3; or
R₁ and R₂ are connected to each other to form -(CH₂)ₚ-, -X(CH₂)₍ₚ₋₁₎-, -(CH₂)₍ₚ₋₁₎X-, -X(CH₂)_{(q-1)}X-, or -(CH₂)_{(q-1)}X(CH₂)_{(q-1)}-, p represents an integer from 2 to 4, q represents an integer from 2 to 3, and X represents O, NH, or S;
R₃ is -H, -CH₃, -(CH₂)ₙCH₃, -OCH₃, -O(CH₂)ₙCH₃, -NHCH₃, -NH(CH₂)ₙCH₃, -(CH₂)ₙSi(CH₃)₃, -(CH₂)ₙN(CH₃)₂, -(CH₂)ₙCH=CH₂, -NO₂, -F, -Cl, -Br, or -I, and n represents an integer from 1 to 3;
R₄ is -H, -CH₃, -OCH₃, -(CH₂)ₙCH₃, -Si(CH₃)₂C(CH₃)₃, -(CH₂)ₙSi(CH₃)₃, -(CH₂)ₙNHCH(CH₃)₂, -CH₂NH(CH₂)ₙCH₃, -CH₂O(CH₂)ₙCH₃, -F, -Cl, -Br, -I, 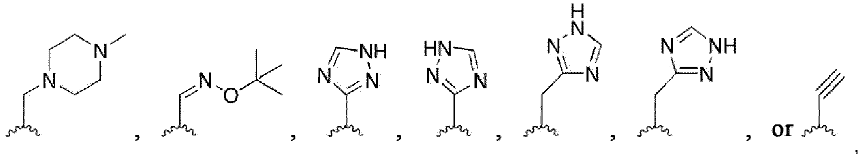 wherein, n represents an integer from 1 to 3; or
R₃ and R₄ are connected to each other to form:

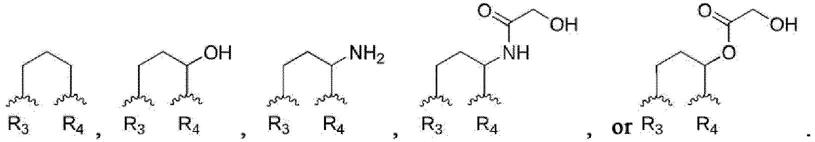

According to an embodiment of the present application, in the general formula Q,
R₁ is -H, -F, -Cl, -Br, or -I;
R₂ is -H, -OH, -CH₃, -(CH₂)ₙCH₃, or 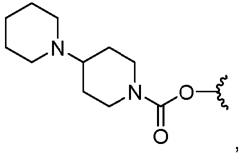 wherein, n represents an integer from 1 to 3; or
R₁ and R₂ are connected to each other to form -X(CH₂)_{(q-1)}X-, q represents an integer from 2 to 3, and X represents O or S;
R₃ is -H, -(CH₂)ₙN(CH₃)₂, -(CH₂)ₙCH=CH₂, or -NO₂, and n represents an integer from 1 to 3;
R₄ is -H, -CH₃, -(CH₂)ₙCH₃, -Si(CH₃)₂C(CH₃)₃, -(CH₂)ₙSi(CH₃)₃, -(CH₂)ₙNHCH(CH₃)₂, 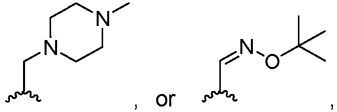 wherein, n represents an integer from 1 to 3; or
R₃ and R₄ are connected to each other to form:

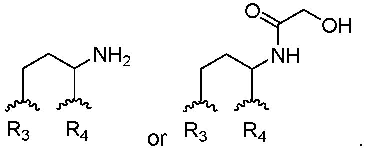

According to an embodiment of the present application, in the general formula Q,
R₁ is -H or -F;
R₂ is -H, -OH, -CH₃, or 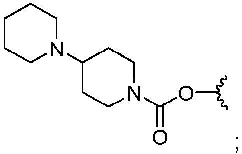 or
R₁ and R₂ are connected to each other to form -O(CH₂)₂O-;
R₃ is -H, -(CH₂)N(CH₃)₂, -CH₂CH=CH₂, or -NO₂;
R₄ is -H, -CH₂CH₃, -Si(CH₃)₂C(CH₃)₃, -(CH₂)₂Si(CH₃)₃, -(CH₂)₂NHCH(CH₃)₂, 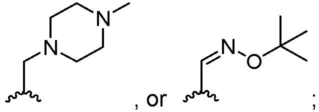 or
R₃ and R₄ are connected to each other to form:

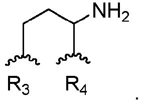

According to an embodiment of the present application, in the general formula Q,
R₁ is -H or -F;
R₂ is -H, -OH, or -CH₃;
R₃ is -H;
R₄ is -CH₂CH₃ or -(CH₂)₂NHCH(CH₃)₂; or
R₃ and R₄ are connected to each other to form:

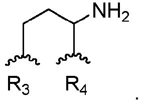

According to an embodiment of the present application, the dideuterated camptothecin derivative is selected from the group consisting of:

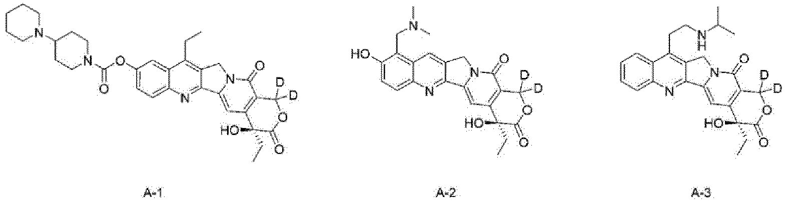

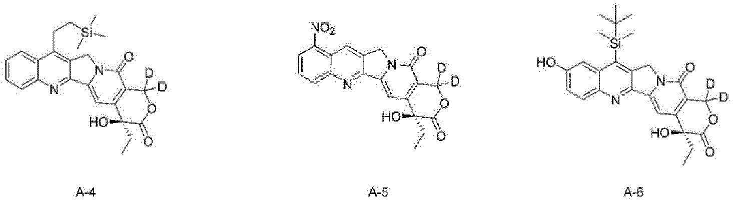

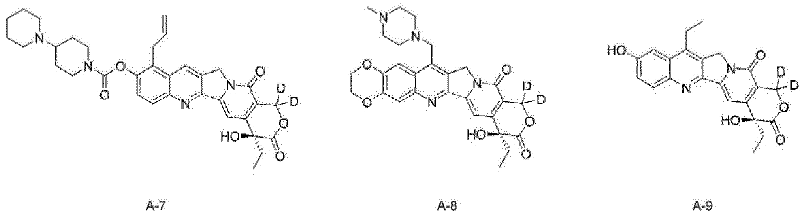

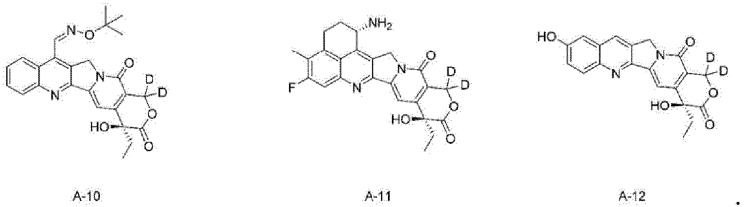

The present application provides a preparation method of the dideuterated camptothecin derivative, wherein the method comprises the following steps: 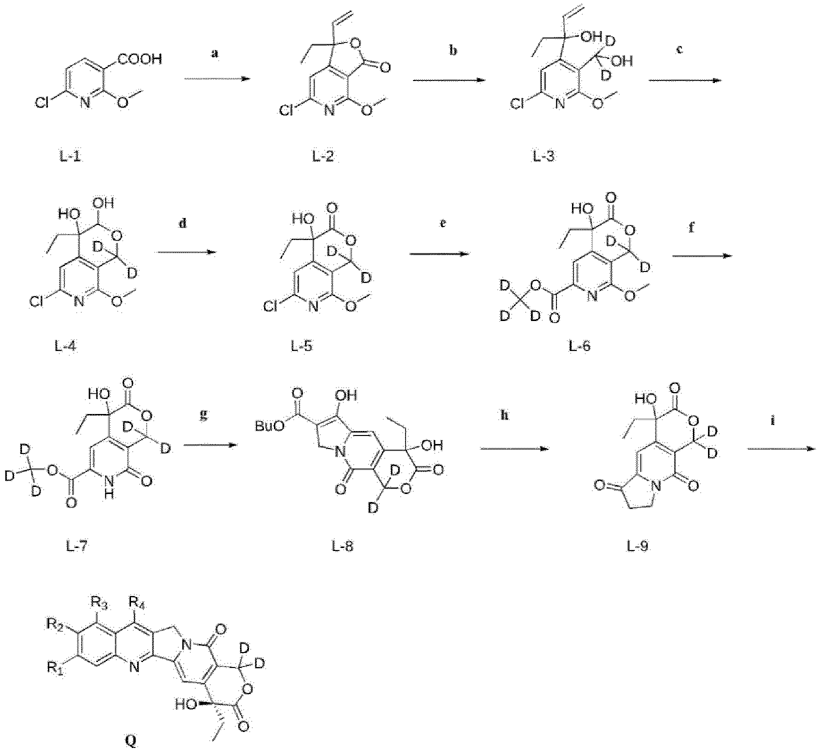
a) reacting compound L-1 with 1-penten-3-one, 2,2,6,6-tetramethylpiperidine, and a lithiation reagent to obtain compound L-2;
b) deuterating compound L-2 with a deuterated reducing agent to obtain compound L-3;
c) subjecting compound L-3 to an ozonization reaction to obtain compound L-4;
d) reacting compound L-4 with 2,2,6,6-tetramethylpiperidine oxide, sodium bicarbonate, potassium bromide, and sodium hypochlorite to obtain compound L-5;
e) reacting compound L-5 with a palladium catalyst, a phosphine ligand, a base, and an alcohol in an atmosphere of carbon monoxide to obtain compound L-6;
f) reacting compound L-6 with trimethylchlorosilane and sodium iodide to obtain compound L-7;
g) reacting compound L-7 with a base and an ester to obtain compound L-8;
h) decarboxylating compound L-8 in toluene as a solvent in the presence of trifluoroacetic acid to obtain compound L-9; and
i) reacting compound L-9 with a polysubstituted benzene ring 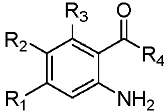 in the presence of a catalyst to obtain compound Q; wherein in the polysubstituted benzene ring, R₁ to R₄ are as defined in the general formula Q above.

According to an embodiment of the present application, the preparation method of the dideuterated camptothecin derivative as shown in general formula Q comprises the following steps:
a) reacting compound L-1 with 1-penten-3-one, 2,2,6,6-tetramethylpiperidine, and a lithiation reagent to obtain compound L-2 at a reaction temperature of -78 °C to -10 °C for a reaction time of 2 to 12 hours; wherein the lithiation reagent is selected from the group consisting of n-butyllithium, isobutyllithium, and lithium diisopropylamide; a solvent selected from the group consisting of tetrahydrofuran, methyltetrahydrofuran, ethyl acetate, and petroleum ether is used; and a reaction molar ratio of compound L-1, 1-penten-3-one, 2,2,6,6-tetramethylpiperidine, and the lithiation reagent is 1 : 2 to 4 : 2 to 4 : 3 to 5;
b) deuterating compound L-2 with a deuterated reducing agent to obtain compound L-3; wherein the deuterated reducing agent is selected from the group consisting of deuterated sodium borohydride, deuterated potassium borohydride, deuterated borane, and deuterated lithium aluminum hydride; a solvent selected from the group consisting of dichloromethane, tetrahydrofuran, methanol, and ethanol is used; a reaction molar ratio of compound L-2 to the deuterated reducing agent is 1 : 1.1 to 2.5; a reaction temperature is 0 °C to 40 °C, preferably 10 °C to 35 °C, more preferably 20 °C to 30 °C; and a reaction time is 6 to 15 hours, preferably 8 to 14 hours;
c) subjecting compound L-3 to an ozonization reaction to obtain compound L-4, wherein a solvent is selected from the group consisting of ethanol, methanol, tetrahydrofuran, and dichloromethane; a reaction temperature is -78 °C; and a reaction time is 15 to 30 minutes;
d) reacting compound L-4 with 2,2,6,6-tetramethylpiperidine oxide, sodium bicarbonate, potassium bromide, and sodium hypochlorite to obtain compound L-5; wherein a reaction molar ratio thereof is 1 : 0.01 to 0.1 : 0.1 to 0.5 : 0.1 to 0.5 : 1 to 5; and a reaction temperature is -10 °C to 10 °C;
e) reacting compound L-5 with a palladium catalyst, a phosphine ligand, a base, and an alcohol in the atmosphere of carbon monoxide to obtain compound L-6; wherein the base is selected from the group consisting of potassium carbonate, sodium carbonate, cesium carbonate, potassium phosphate, and triethylamine; the palladium catalyst is selected from the group consisting of palladium acetate, bistriphenylphosphine palladium dichloride, 1,1-bis(diphenylphosphine)ferrocene palladium dichloride, and triphenylphosphine palladium; the alcohol is deuterated methanol or deuterated ethanol; the phosphine ligand is 1,3-bis(diphenylphosphine)propane; a reaction molar ratio of compound L-5, the palladium catalyst, the phosphine ligand, and the base is 1 : 0.01 to 0.1 : 0.01 to 0.1 : 1.5 to 3; a solvent is N,N-dimethylformamide; a reaction temperature is 60 °C to 120 °C; and a reaction time is 6 to 48 hours;
f) reacting compound L-6 with trimethylchlorosilane and sodium iodide to obtain compound L-7; wherein a reaction molar ratio of compound L-6, trimethylchlorosilane, and sodium iodide is 1 : 2 to 3 : 2 to 3; a solvent is selected from the group consisting of acetonitrile, tetrahydrofuran, dichloromethane, and ethyl acetate; a reaction time is 12 to 24 hours; and a reaction temperature is room temperature;
g) reacting compound L-7 with a base and an ester to obtain compound L-8, wherein a reaction molar ratio of compound L-7, the base, and the ester is 1 : 2 to 4 : 2 to 10; a reaction time is 12 to 48 hours; a reaction temperature is 50 °C; and compound L-8 is a five-membered ring compound; wherein the base is selected from the group consisting of potassium carbonate, cesium carbonate, sodium carbonate, and sodium ethoxide; the ester is selected from the group consisting of methyl acrylate, ethyl acrylate, tert-butyl acrylate, and n-butyl acrylate; and a solvent is N,N-dimethylformamide or dimethyl sulfoxide;
h) decarboxylating compound L-8 in toluene as a solvent in the presence of trifluoroacetic acid to obtain compound L-9; wherein a reaction temperature is 80 °C to 140 °C, and a reaction time is 2 to 6 hours; and
i) reacting compound L-9 with a polysubstituted benzene ring 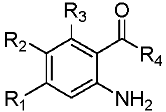 in the presence of a catalyst to obtain compound Q; wherein a reaction molar ratio of compound L-9, the polysubstituted benzene ring and the catalyst is 1 : 1 : 0.2 to 0.6; a solvent selected from the group consisting of tetrahydrofuran, ethanol, and toluene is used for reaction; the catalyst is selected from anhydrous p-toluene sulfonic acid, pyridinium p-toluenesulfonate, antimony trichloride, sulfuric acid, and concentrated hydrochloric acid; a reaction temperature is 80 °C to 140 °C; a reaction time is 12 to 48 hours; and in the polysubstituted benzene ring, R₁ to R₄ are as defined in the general formula Q above.

According to an embodiment of the present application, a preparation method of compound L-3 is provided, wherein the method comprises the following step: 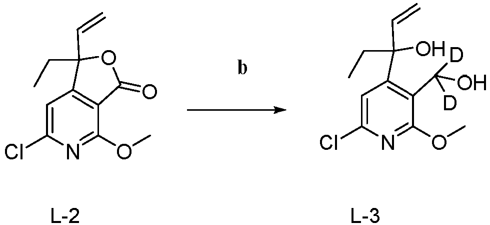
reacting compound L-2 with a deuterated reducing agent in solvent I to obtain compound L-3,
wherein, the deuterated reducing agent is selected from the group consisting of deuterated sodium borohydride, deuterated potassium borohydride, deuterated borane, and deuterated lithium aluminum hydride; and
the solvent I is selected from the group consisting of dichloromethane, tetrahydrofuran, methanol, and ethanol, preferably an anhydrous solvent of the following solvents: dichloromethane, tetrahydrofuran, methanol, and ethanol.

In an embodiment of the present application, the reaction molar ratio of compound L-2 to the deuterated reducing agent is 1 : 1.1 to 2.5.

In an embodiment of the present application, the reaction temperature of compound L-2 and the deuterated reducing agent is 0 °C to 40 °C, preferably 10 °C to 35 °C, more preferably 20 °C to 30 °C.

In an embodiment of the present application, the reaction time of compound L-2 reacting with the deuterated reducing agent is 6 to 15 hours, preferably 8 to 14 hours.

In an embodiment of the present application, the preparation method of compound L-3 comprises the following steps: adding compound **L-2** to anhydrous ethanol, cooling down the system to 0 °C, adding deuterated sodium borohydride, stirring for 15 minutes, then heating the reaction system to room temperature, and reacting for 12 hours; after completion of the reaction as monitored by TLC, adding hydrochloric acid, extracting the system by adding dichloromethane, combining the organic phases, washing with saturated sodium bicarbonate, washing with saturated saline, drying with anhydrous sodium sulfate, concentrating the system under vacuum, and purifying by column chromatography to obtain compound L-3.

In an embodiment of the present application, the preparation method of compound L-3 comprises the following steps: adding 21 g of compound **L-2** to 400 mL of anhydrous ethanol, cooling down the system to 0 °C, adding 14.21 g of deuterated sodium borohydride, stirring for 15 minutes, then heating the reaction system to room temperature, and reacting for 12 hours; after completion of the reaction as monitored by TLC, adding 30 mL of 1 N hydrochloric acid, extracting the system by adding dichloromethane, combining the organic phases, washing with saturated sodium bicarbonate, washing with saturated saline, drying with anhydrous sodium sulfate, concentrating the system under vacuum, and purifying by column chromatography to obtain compound L-3.

According to an embodiment of the present application, a preparation method of compound L-6 is provided, wherein the method comprises the following step: 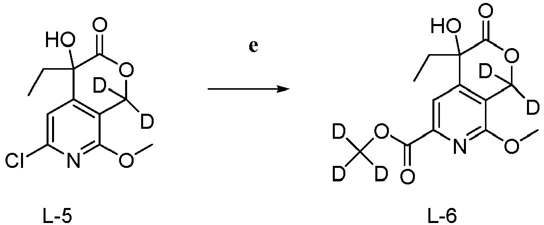
e) reacting compound L-5 with a palladium catalyst, a phosphine ligand, a base, and an alcohol in an atmosphere of carbon monoxide to obtain compound L-6,
wherein, the base is selected from the group consisting of potassium carbonate, sodium carbonate, cesium carbonate, potassium phosphate, and triethylamine; the palladium catalyst is selected from the group consisting of palladium acetate, bistriphenylphosphine palladium dichloride, 1,1-bis(diphenylphosphine)ferrocene palladium dichloride, and triphenylphosphine palladium; the alcohol is deuterated methanol or deuterated ethanol; the phosphine ligand is 1,3-bis(diphenylphosphine)propane; a reaction molar ratio of compound L-5, the palladium catalyst, the phosphine ligand and the base is 1 : 0.01 to 0.1 : 0.01 to 0.1 : 1.5 to 3; a solvent is N,N-dimethylformamide; a reaction temperature is 60 °C to 120 °C; and a reaction time is 6 to 48 hours.

### BENEFICIAL EFFECTS

The compound of the present application has more excellent tumor inhibitory activity, and has more excellent elimination rate and elimination half-life. When the compound is used as a payload to form an ADC drug, if it is shed from the resulting ADC drug *in vivo,* it can be rapidly metabolized and eliminated, thereby reducing the toxicity of the drug *in vivo.* In addition, deuteration technology is an important part of modern medical imaging technology. Based on deuteration on the parent core structure of camptothecin, camptothecin and derivatives thereof can be used in tracer technology for medical research.

In the present application, the concentration unit "M" refers to mol/L, e.g., 1 M=1 mol/L; and equivalent concentration (N) refers to the concentration of the solution expressed by the gram equivalent number of solutes contained in 1 liter of solution, which is called equivalent concentration, denoted by the symbol N. As for hydrochloric acid, in which one hydrogen ion is released, the equivalent concentration is the same as the molar concentration. In the case of sulfuric acid, the equivalent concentration is equal to 0.5 times the molar concentration.

The confirmation of deuterated position of deuterated compound of the present application (e.g. compound L3) is known to those skilled in the art, which can be confirmed, for example, by comparing with ¹H-NMR data of a corresponding non-deuterated compound. The steric configuration of the compound of the present application (e.g. compound L3, compound A-3, compound A-9, and compound A-11) can be determined by comparing the deuterated compound with the corresponding non-deuterated single isomer in terms of the HPLC retention time or the specific rotation.

### DETAILED DESCRIPTION

Those skilled in the art understand that the materials used in the following, unless otherwise specified, are known in the art and are commercially available or can be obtained by those skilled in the art on the basis of published literature or conventional methods. Unless otherwise indicated, all the reactions of the present application are carried out under continuous magnetic agitation in a dry atmosphere of nitrogen or argon, the solvent being a dry solvent, in which: (i) the temperature is expressed in degrees Celsius (°C), the operation is carried out at room temperature, and the room temperature generally refers to 15 °C to 35 °C, preferably 20 °C to 30 °C, more preferably 20 °C to 25 °C; (ii) solvent is removed by evaporation under reduced pressure using rotary evaporator, bath temperature is not higher than 60 °C; (iii) the reaction process is monitored by thin layer chromatography (TLC); and (iv) the final product has satisfactory hydrogen nuclear magnetic resonance spectroscopy (1H-NMR) and/or mass spectrometry (MS) data.

The dideuterated camptothecin derivative and the preparation method therefor of the present application are described in more detail in the following examples, but the examples do not mean a limitation to the scope of the present application.

### Example 1

### 1.1 Preparation of compound L-2

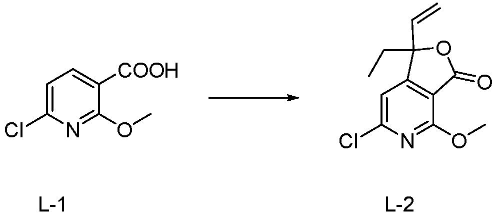

Into a 3 L three-necked flask, 86.4 mL of 2,2,6,6-tetramethylpiperidine was added, and then 1 L of anhydrous tetrahydrofuran was added. The system was cooled down to -78 °C and reacted for 15 minutes. 272 mL of 2.5 M n-butyl lithium in petroleum ether was added, and the system reacted for 30 minutes. 32 g of compound L-1 was dissolved in 200 mL of anhydrous tetrahydrofuran. The mixture was added to the system, and the reaction was carried out for 1 hour. 50 mL of 1-penten-3-one was dissolved in 200 mL of anhydrous tetrahydrofuran, the mixture was added to the system, and the reaction was carried out for 1 hour. After completion of the reaction as monitored by TLC, 640 mL of 4 N hydrochloric acid was added, and the system was extracted with ethyl acetate. The organic phases were combined, washed with saturated sodium bicarbonate, then washed with saturated saline, and dried by anhydrous sodium sulfate. The system was concentrated under vacuum, and then purified by column chromatography to obtain 21 g of compound L-2 as a white solid with a yield of 49%. ESI-MS m/z: 253.68, [M+H]⁺.

¹H NMR (400 MHz, CDCl₃) δ 6.99 (s, 1H), 6.13 (dd, *J* = 17.3, 10.6 Hz, 1H), 5.29 (d, *J* = 7.0 Hz, 1H), 5.26 (s, 1H), 3.99 (s, 3H), 2.73 (s, 2H), 2.04 (dt, *J =* 14.8, 7.3 Hz, 1H), 1.98 - 1.87 (m, 1H), 0.91 (t, *J* = 7.3 Hz, 3H).

### Example 2

### 1.2 Preparation of compound L-3

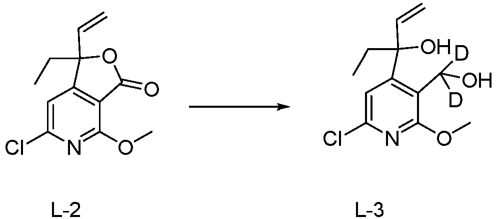

Into a 1 L three-necked flask, 21 g of compound L-2 was added, and then 400 mL of anhydrous ethanol was added. The system was cooled down to 0 °C, added with 14.21 g of deuterated sodium borohydride, stirred for 15 minutes, then heated to room temperature, and reacted for 12 hours. After completion of the reaction as monitored by TLC, 30 mL of 1 N hydrochloric acid was added, and the system was extracted with dichloromethane. The organic phases were combined, washed with saturated sodium bicarbonate, then washed with saturated saline, and dried by anhydrous sodium sulfate. The system was concentrated under vacuum, and then purified by column chromatography to obtain 17 g of compound L-3 as a transparent thick liquid with a yield of 80%. ESI-MS m/z: 259.73, [M+H]⁺. The purity of the deuterated product was confirmed to be more than 99% by comparing with the ¹H-NMR data of the corresponding L-3 non-deuterated compound.

¹H NMR (400 MHz, CDCl₃) δ 6.99 (s, 1H), 6.13 (dd, *J=* 17.3, 10.6 Hz, 1H), 5.29 (d, *J=* 7.0 Hz, 1H), 5.26 (s, 1H), 3.99 (s, 3H), 2.73 (s, 2H), 2.04 (dt, *J =* 14.8, 7.3 Hz, 1H), 1.98 - 1.87 (m, 1H), 0.91 (t, *J =* 7.3 Hz, 3H).

### Example 3

### 1.3 Preparation of compound L-4

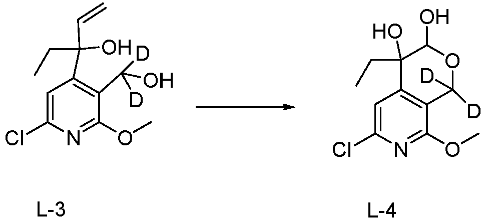

Into a 250 mL round-bottom flask, 2 g of compound L-3 was added, and then 100 mL of dichloromethane was added. The system was cooled down to -78 °C, and ozone was introduced under this condition. After completion of the reaction as monitored by TLC, 0.5 mL of dimethyl sulfide was added. The system was heated to room temperature and stirred for 30 minutes. The system was concentrated under vacuum, and then purified by column chromatography to obtain 1.9 g of compound **L-4** as a white solid with a yield of 95%. The product was directly used for the next reaction step without further purification by separation. ESI-MS m/z: 261.70, [M+H]⁺.

¹H NMR (400 MHz, CDCl₃) δ 7.15 (s, 1H), 5.19 (d, *J =* 4.8 Hz, 1H), 3.96 (s, 3H), 3.21 (d, *J =* 4.8 Hz, 1H), 2.64 (s, 1H), 1.80 (q, *J =* 7.5 Hz, 2H), 0.92 (t, *J =* 7.5 Hz, 3H).

### Example 4

### 1.4 Preparation of compound L-5

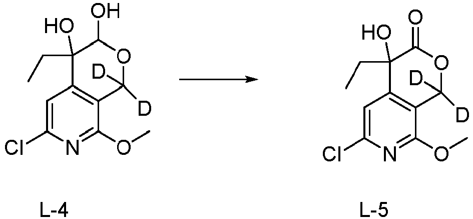

Into a 1 L single-necked flask, 12 g of compound L-4 was added, and then 240 mL of dichloromethane was added. The system was stirred for 30 minutes, and cooled down to 0 °C. The system was added with 286 mg of 2,2,6,6-tetramethylpiperidine oxide, 616 mg of sodium bicarbonate, 654 mg of potassium bromide, and 18 mL of water, and stirred for 15 minutes. Then 120 mL of sodium hypochlorite solution (> 7.5%wt) was added, and the reaction was carried out for 30 minutes. After completion of the reaction as monitored by TLC, 12 g of sodium bisulfite was added, and the system was extracted with dichloromethane. The organic phases were combined, washed with water, then washed with saturated saline, and dried by anhydrous sodium sulfate. The system was concentrated under vacuum, and then purified by column chromatography to obtain 6.6 g of compound **L-5** as a white solid with a yield of 55%. ESI-MS m/z: 259.68, [M+H]⁺.

¹H NMR (400 MHz, CDCl₃) δ 7.20 (s, 1H), 4.00 (s, 3H), 3.64 (s, 1H), 1.79 (q, *J =* 7.4 Hz, 2H), 0.97 (t, *J =* 7.4 Hz, 3H).

### Example 5

### 1.5 Preparation of compound L-6

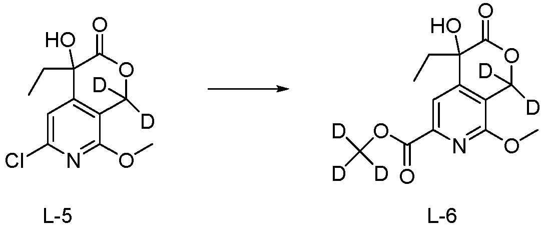

In the atmosphere of carbon monoxide, 3 g of compound **L-5** was added into a 100 mL three-necked flask, and 286 mg of 1,3-bis(diphenylphosphine)propane, 2.4 g of potassium carbonate, 129 mg of palladium acetate, 15 mL of N,N-dimethylformamide, and 30 mL of deuterated methanol were added. The system was heated to 65 °C and the reaction was carried out for 12 hours. After completion of the reaction as monitored by TLC, 20 mL of 1 N hydrochloric acid was added, and the system was extracted with ethyl acetate. The organic phases were combined, washed with saturated sodium bicarbonate, then washed with saturated saline, and dried by anhydrous sodium sulfate. The system was concentrated under vacuum, and then purified by column chromatography to obtain 1.33g of compound L-6 as a white solid with a yield of 40%. ESI-MS m/z: 286.29, [M+H]⁺.

¹H NMR (400 MHz, CDCl₃) δ 7.98 (d, *J =* 2.0 Hz, 1H), 4.09 (d, *J =* 2.0 Hz, 3H), 3.71 (d, *J =* 2.6 Hz, 1H), 1.88 - 1.75 (m, 2H), 0.96 (td, *J =* 7.3, 1.9 Hz, 3H).

### Example 6

### 1.6 Preparation of compound L-7

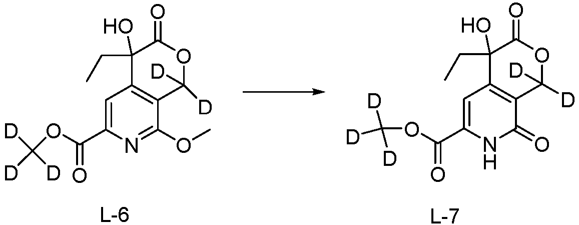

Into a 50 mL double-necked flask, 1 g of compound L-6 was added, and 1.05 g of sodium iodide and 10 mL acetonitrile were added. The system was cooled down to 0 °C and stirred for 30 minutes. The system was added with 0.89 mL of trimethylchlorosilane, heated to room temperature, and reacted for 12 hours. After completion of the reaction as monitored by TLC, 40 mL of water and 1 mL of saturated sodium bisulfite solution were added. The system was stirred for 1 hour, extracted with dichloromethane, and dried with anhydrous sodium sulfate. The system was concentrated under vacuum, and then purified by column chromatography to obtain 800 mg of compound **L-7** as a white solid with a yield of 84%. ESI-MS m/z: 272.27, [M+H]⁺.

¹H NMR (400 MHz, CDCl₃) δ 10.03 (s, 1H), 7.32 (s, 1H), 3.90 (s, 1H), 1.81 (dd, *J =* 14.5, 7.2 Hz, 2H), 0.99 (t, *J =* 7.4 Hz, 3H).

### Example 7

### 1.7 Preparation of compound L-8

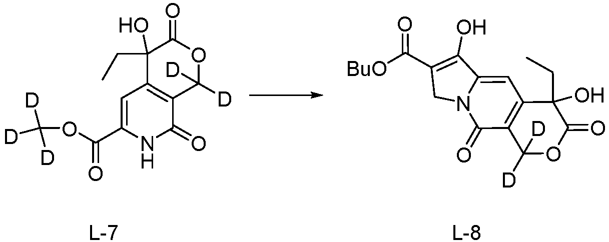

Into a 25 mL double-necked flask, 620 mg of compound L-7 was added, and 1.48 g of cesium carbonate, 8 mL of dimethyl sulfoxide and 1.67 mL of tert-butyl acrylate were added. The system was heated to 50 °C and reacted for 12 hours. After completion of the reaction as monitored by TLC, 1 mL of concentrated hydrochloric acid was added, and then 40 mL water was added. The system was extracted with dichloromethane, and dried with anhydrous sodium sulfate. The system was concentrated under vacuum, and then purified by column chromatography to obtain 468 mg of compound **L-8** as a light brown solid with a yield of 55%. ESI-MS m/z: 365.38, [M+H]⁺.

### Example 8

### 1.8 Preparation of compound L-9

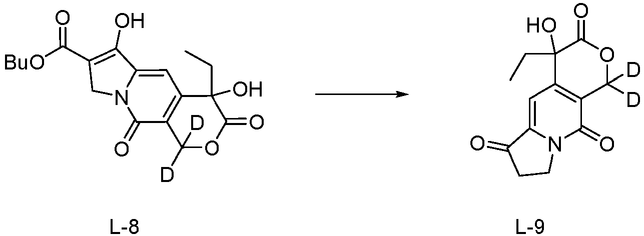

Into a 10 mL single-necked flask, 200 mg of compound L-8 was added, and 5 mL of toluene and 0.5 mL of trifluoroacetic acid were added. The system was heated to 110 °C and reacted for 2 hours. After completion of the reaction monitored by TLC, the system was concentrated under vacuum, and then purified by column chromatography to obtain 140 mg of compound L-9 as an orange solid with a yield of 98%. ESI-MS m/z: 265.26, [M+H]⁺.

### Example 9

### 1.9 Preparation of compound (A-9)

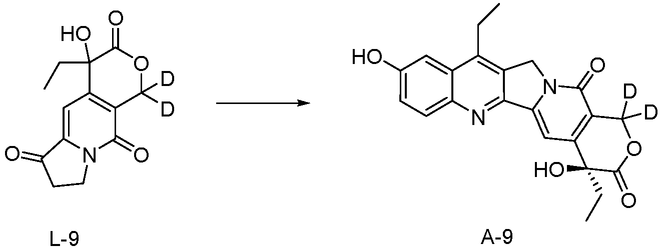

Into a 10 mL single-necked flask, 140 mg of compound L-9 was added, and 89 mg of 1-(2-amino-5-hydroxyphenyl)propan-1-one, 2.5 mL of toluene, 2.5 mL of acetic acid, and 41 mg of pyridinium p-toluenesulfonate were added. The system was heated to 110 °C and reacted for 18 hours. After completion of the reaction as monitored by TLC, the system was concentrated under vacuum, and then purified by column chromatography (IH 4.6*100 mm, 5 µm chiral column, 30% methanol/n-hexane) to obtain 80 mg of compound **A-9,** corresponding to the second isomer absorption peak, as a light yellow solid with a yield of 37%. ESI-MS m/z: 394.42, [M+H]⁺.

¹H NMR (500 MHz, DMSO-d6) δ 0.88 (t, J = 7.25 Hz, 3H),1.32 (t, J = 7.5 Hz, 3H), 1.85 (m, 2H), 3.11 (q, J = 7.5 Hz, 2H), 5.26(s, 2H), 6.48(s, 1H), 7.23 (s, 1H), 7.41 (d, J = 10.0 Hz,2H), 8.01 (d, J = 10.0 Hz, 1H), 10.3 (s, 1H).

### Example 10

### 2.1 Preparation of compound (A-3)

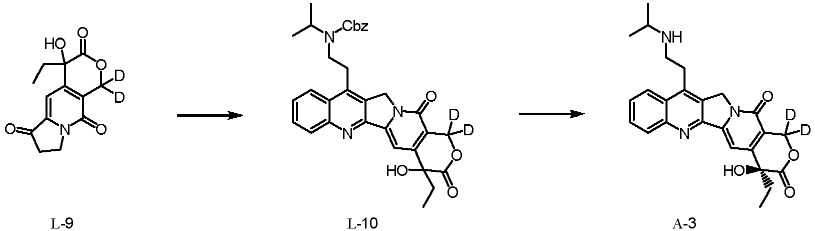

Into a 10 mL single-necked flask, 140 mg of compound L-9 was added, and 180 mg of benzyl (3-(2-aminophenyl)-3-oxo-propyl)(isopropyl)carbamate, 2.5 mL of toluene, 2.5 mL of acetic acid, and 41 mg of pyridinium p-toluenesulfonate were added. The system was heated to 110 °C and reacted for 18 hours. After completion of the reaction as monitored by TLC, the system was concentrated under vacuum, and then purified by column chromatography to obtain 240 mg of intermediate compound **L-10** as a golden solid with a yield of 80%.

Into a 25 mL single-necked flask, 240 mg of intermediate compound **L-10** was added, and 105 mL of acetic acid and 105 mg of 10% palladium carbon were added. The atmosphere of the system was replaced with hydrogen. The system was reacted at room temperature for 24 hours. After completion of the reaction as monitored by TLC, the system was concentrated under vacuum, and then purified by column chromatography (IH 4.6*100 mm, 5 µm chiral column, 30% methanol/n-hexane) to obtain 71 mg of compound **A-3,** corresponding to the second isomer absorption peak, as a golden solid with a yield of 39%. ESI-MS m/z: 435.52, [M+H]⁺.

¹H NMR (400 MHz, DMSO) δ 9.23 (s, 2H), 8.45 (d, *J =* 8.4 Hz, 1H), 8.21 (d, *J* = 8.4 Hz, 1H), 7.90 (t, *J* = 7.5 Hz, 1H), 7.79 (t, *J =* 7.5 Hz, 1H), 7.36 (s, 1H), 5.43 (s, 2H), 3.63 (d, *J =* 8.5 Hz, 2H), 3.42 - 3.34 (m, 1H), 3.21 (s, 2H), 1.95 - 1.81 (m, 2H), 1.28 (d, *J =* 6.2 Hz, 6H), 0.88 (t, *J =* 7.0 Hz, 3H).

### Example 11

### 3.1 Preparation of compound (A-11)

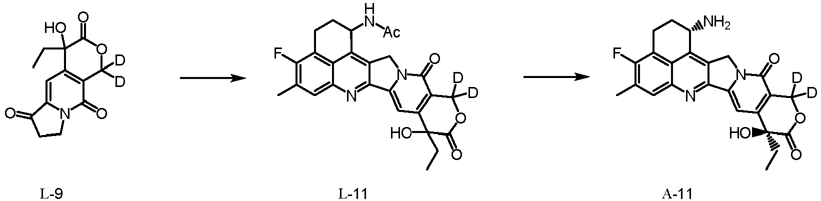

Into a 25 mL single-necked flask, 300 mg compound L-9 was added, and 266 mg of N-(8-amino-6-fluoro-5-methyl-1-oxo-1,2,3,4-tetrahydronaphthalen-2-yl)acetamide, 9 mL of toluene, 770 mL of o-cresol, and 133 mg of pyridinium p-toluenesulfonate were added. The system was heated to 110 °C and reacted for 18 hours. After completion of the reaction as monitored by TLC, the system was concentrated under vacuum, and then purified by column chromatography to obtain 443 mg of intermediate compound L-11 as a yellow-green solid with a yield of 81%.

Into a 25 mL single-necked flask, 443 mg of intermediate compound L-11 was added, and 7 mL of pure water and 2.2 mL of methanesulfonic acid were added. The atmosphere of the system was replaced with nitrogen. The system was heated to 85 °C and reacted for 10 hours. After completion of the reaction as monitored by TLC, the system was concentrated under vacuum, and then purified by column chromatography (chiral column CHIRALPAK IK-3, 250 mm×4.6 mm, 3 µm, mobile phase A = ethanol (containing 0.1% diethylamine), mobile phase B = n-hexane (containing 0.1% diethylamine)) to obtain 141 mg of compound A-11, corresponding to the first isomer absorption peak, as a yellow-green solid with a yield of 35%. ESI-MS m/z: 437.47, [M+H]+.

¹H NMR (400 MHz, D₂O) δ 7.20 - 7.06 (m, 2H), 5.42 - 5.14 (m, 5H), 3.30 (dd, J = 18.1, 4.0 Hz, 1H), 3.06 - 2.91 (m, 1H), 2.69 (s, 2H), 2.62 - 2.47 (m, 1H), 2.18 (s, 3H), 1.80 (q, J = 7.3 Hz, 2H), 0.79 (t,J = 7.4 Hz, 3H).

### Biological Activity Assay

### Test Example 1: HCT-116 cell inhibitory activity test

HCT116 cells (purchased from ATCC) were cultured in a 5% CO₂ incubator at 37 °C using DMEM culture medium (purchased from Gibco) supplemented with 10% fetal bovine serum and 1% penicillin-streptomycin solution. The cells were seeded in 96-well plates at 3000 cells per well and cultured overnight. Different concentrations of drugs were respectively added, and the cells were placed in a normal incubator (21 % O₂, 5% CO₂, and 74% N₂) and cultured for 72 h. 100 µL of culture medium was removed. 10 µL of MTT solution (5 mg/mL) was added. Then the cells were cultured in the incubator for 4 hours. 50 µL of triple combination solution (10% SDS, 5% isobutanol, and 0.012 mol/L HCl) was added. The cells were placed in the incubator overnight. OD values were measured at 540 nm wavelength, and median inhibition concentration IC₅₀ of the drug on tumor cell growth was calculated (n=3). The result is as shown in the following Table 1. Compounds with activity designated as "A" provide IC₅₀≤10nM. Compounds with activity designated as "B" provide IC₅₀ of 10 nM to 100 nM. Compounds with activity designated as "C" provide IC₅₀ of 100 nM to 1000 nM. Compounds with activity designated as "D" provide IC₅₀ of 1000 nM to 10000 nM. Titer (nM); Efficacy (IC₅₀).

**Table 1**

| **Compound No.** | IC₅₀ **Activity** |
|---|---|
| **A-3** | A |
| **A-9** | A |
| **A-11** | A |

The experimental data show that compounds A-3, A-9, and A-11 of the present application have good HCT-116 cell inhibitory activity.

### Test Example 2. Determination of inhibitory activity of MDA-MB-468 and other cells

MDA-MB-468 cells (purchased from ATCC) were cultured in L-15 culture medium (purchased from Sigma-Aldrich), NCI-N87 cells (purchased from ATCC) were cultured in RPMI-1640 culture medium (purchased from Gibco), and HT29 cells (purchased from ATCC) were cultured in McCoy's 5A culture medium (purchased from Gibco), each of which was supplemented with 10% fetal bovine serum and 1% penicillin-streptomycin solution, and placed in a 5% CO₂ incubator at 37°C. The cells were seeded in 96-well plates at 3000 cells per well and cultured overnight. Different concentrations of drugs were respectively added, and the cells were placed in a normal incubator (21% O₂, 5% CO₂, and 74% N₂) and cultured for 72 h. 100 µL of culture medium was removed. 10 µL of MTT solution (5 mg/mL) was added. Then the cells were cultured in the incubator for 4 hours. 50 µL of triple combination solution (10% SDS, 5% isobutanol, and 0.012 mol/L HCl) was added. The cells were placed in the incubator overnight. OD values were measured at 540 nm wavelength, and median inhibition concentration IC₅₀ of the drug on tumor cell growth was calculated (n=3). The result is as shown in the following Table 2.

**Table 2**

| **Compound No.** | **MDA-MB-468** | | **NCI-N87** | | **HT29** | |
|---|---|---|---|---|---|---|
| | **IC₅₀ (nM)** | **Inh.max (%)** | **IC₅₀ (nM)** | **Inh.max (%)** | **IC₅₀ (nM)** | **Inh.max (%)** |
| A-3 | A | >80 | B | >80 | B | >90 |
| A-9 | A | >80 | A | >80 | A | >90 |
| A-11 | A | >80 | A | >70 | A | >90 |

Experimental data show that compounds A-3, A-9, and A11 have good inhibitory effects on breast cancer cell MDA-MB-468, human gastric cancer cell NCI-N87, and human colon cancer cell HT29, and are superior to non-deuterated Belotecan, SN-38 and exatecan. This indicats that replacing hydrogen atom at specific positions with deuterium atom can improve the antitumor effect of the drug.

### Test Example 3: Pharmacokinetic test - mice

Experimental objective: To evaluate the pharmacokinetic characteristics of the compound of the present application in mice.

Balb/c mice (purchased from Beijing Vital River) were randomly divided, 12 mice/group, half male and half female, and were injected once with a sample (10% DMSO-90% (20% 2-hydroxypropyl-β-cyclodextrin-physiological saline), diluted according to the dose). Cross samples of plasma were respectively taken before administration, and at 5 min, 30 min, 1 h, 2 h, 4 h, 6 h, 8 h, 10 h, and 24 h after administration. The sample concentration in plasma was determined by LC-MS method, and pharmacokinetic parameters were calculated (see Table 3).

**Table 3 Pharmacokinetic characteristics of the compound of the present application in mice**

| Sample No. | Mice PK (IV, 3 mpk) | | | | |
|---|---|---|---|---|---|
| | T_{1/2} (h) | Cₘₐₓ (ng/ml) | AUCinf (h*ng/ml) | Vz (ml/kg) | Cl (ml/h/kg) |
| A-11 | 1.19 | 902.0 | 321.2 | 15969.9 | 9338.8 |

The experimental results show that the compound has shorter half-life and faster clearance rate *in vivo,* and has better safety *in vivo.*

### Test Example 4. Pharmacokinetic test - rats

Experimental objective: To evaluate the pharmacokinetic characteristics of the compound of the present application in rats.

SD rats (Beijing Vital River Laboratory Animal Technology Co., Ltd.) were randomly divided, 6 rats/group, half male and half female, and were injected once with a sample (10% DMSO-90% (20% 2-hydroxypropyl-β-cyclodextrin-physiological saline), diluted according to the dose). Plasma samples were respectively taken before administration, and at 5 min, 30 min, 1 h, 2 h, 4 h, 6 h, 8 h, 10 h, and 24 h after administration. The sample concentration in plasma was determined by LC-MS method, and pharmacokinetic parameters were calculated (see Table 4).

**Table 4 Pharmacokinetic characteristics of the compound of the present application in SD rats**

| Sample No. | Rat PK (IV, 3 mpk) | | | | |
|---|---|---|---|---|---|
| | T_{1/2} (h) | Cₘₐₓ (ng/ml) | AUCinf(h*ng/ml) | Vz (ml/kg) | Cl (ml/h/kg) |
| A-9 | 1.35 | 288.2 | 99.5 | 19546.9 | 10045.98 |
| A-11 | 0.91 | 215.0 | 134.9 | 30087.8 | 22888.61 |

The experimental results show that the compounds have shorter half-life and faster clearance *in vivo,* and thus have good safety *in vivo,* which are superior to the corresponding non-deuterated compounds.

### Test Example 5. Evaluation of liver microsomal stability in vitro

Each incubation system containing phosphoric acid buffer (PBS, pH 7.4), liver microsome protein, sample to be tested (acetonitric solution), and NADPH was incubated in a water bath at 37 °C. The reaction was terminated by adding the same volume of ice-cold acetonitrile (containing internal standard) after reacting for 0.5 min, 5 min, 15 min, 30 min, and 60 min, respectively. The sample solution was centrifuged. 100 µL of supernatant was taken, and added with 100 µL of ultra-pure water. The remaining content of the substrate of primary form was determined by LC-MS/MS method. The negative control was incubated with heat-inactivated liver microsomes of the corresponding species.

The experimental results show that the compounds of the present application have excellent liver microsomal stability *in vitro,* which are superior to the corresponding non-deuterated compounds.

### Test Example 6. Plasma stability in vitro

398 µL of plasma was preheated to 37 °C and incubated for 15 minutes. 2 µL of sample solution was added to the plasma with a final sample concentration of 5 µM. The above solution was co-incubated at 37 °C. 50 µL of solution was taken at 0 min, 15 min, 30 min, 60 min, and 120 min, respectively, and was added to 450 µL of ice-cold acetonitrile (containing internal standard). After vortexing for 10 minutes, the solution was centrifuged, and the remaining content of the substrate of primary form was determined by LC-MS/MS method.

### Test Example 7. Toxicity test of repeated administration in rats

The possible toxic reactions and severity thereof of the drug on SD rats were observed after continuous intravenous administration for 7 days. The toxicity of the compound in rodents was preliminarily evaluated. SD rats (Beijing Vital River Laboratory Animal Technology Co., Ltd.) were randomly divided, 6 rats/group, half male and half female, and injected with samples (preparation: 10% DMSO-90% (20% 2-hydroxypropyl-β-cyclodextrin-physiological saline), diluted according to the dose) once a day for 7 consecutive days. Weight and food intake were monitored every two days, and the status of the animals was observed. Blood samples were taken 24 h after the last dose for hematology, blood biochemistry and coagulation indexes.

The experimental results show that compounds A-3, A-9, and A-11 of the present application have better safety.

## Claims

1. A dideuterated camptothecin derivative as shown in general formula Q, a stereoisomer thereof, a pharmaceutically acceptable salt thereof, or a prodrug thereof, 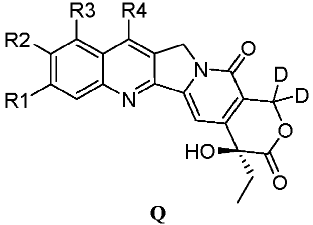 wherein:
R₁ is -H, -CH₃, -(CH₂)ₙCH₃, -OCH₃, -O(CH₂)ₙCH₃, -F, -Cl, -Br, or -I, and n represents an integer from 1 to 3;
R₂ is -H, -OH, -NH₂, -F, -Cl, -Br, -I, -CH₃, -OCH₃, -(CH₂)ₙCH₃, -(CH₂)ₙSi(CH₃)₃, -(CH₂)ₙNH(CH₃)₂, -O(CH₂)ₙCH₃, -O(CH₂)ₙNH₂, -NH(CH₂)ₙNH₂, -(OCH₂CH₂)ₙNH₂, or 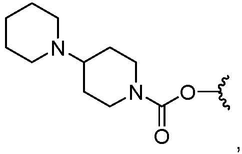 wherein, n represents an integer from 1 to 3; or
R₁ and R₂ are connected to each other to form -(CH₂)ₚ-, -X(CH₂)₍ₚ₋₁₎-, -(CH₂)₍ₚ₋₁₎X-, -X(CH₂)_{(q-1)}X-, or -(CH₂)_{(q-1)}X(CH₂)_{(q-1)}, p and q each independently represents an integer from 2 to 4, and X represents O, NH, or S;
R₃ is -H, -CH₃, -(CH₂)ₙCH₃, -OCH₃, -O(CH₂)ₙCH₃, -NHCH₃, -NH(CH₂)ₙCH₃, -(CH₂)ₙSi(CH₃)₃, -(CH₂)ₙN(CH₃)₂, -(CH₂)ₙCH=CH₂, -NO₂, -F, -Cl, -Br, or -I, and n represents an integer from 1 to 3;
R₄ is -H, -CH₃, -OCH₃, -(CH₂)ₙCH₃, -Si(CH₃)₂C(CH₃)₃, -(CH₂)ₙSi(CH₃)₃, -(CH₂)ₙNHCH(CH₃)₂, -CH₂NH(CH₂)ₙCH₃, -CH₂O(CH₂)ₙCH₃, -F, -Cl, -Br, -I, 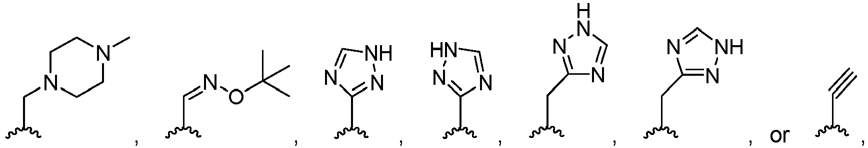 wherein, n represents an integer from 1 to 3; or
R₃ and R₄ are connected to each other to form:

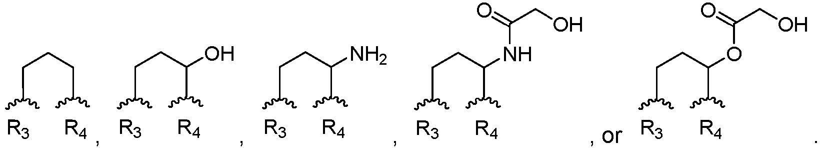

2. The dideuterated camptothecin derivative, the stereoisomer thereof, the pharmaceutically acceptable salt thereof, or the prodrug thereof according to claim 1, 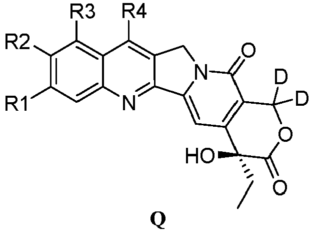 wherein:
R₁ is -H, -CH₃, -(CH₂)ₙCH₃, -OCH₃, -O(CH₂)ₙCH₃, -F, -Cl, -Br, or -I, and n represents an integer from 1 to 3;
R₂ is -H, -OH, -NH₂, -F, -Cl, -Br, -I, -CH₃, -OCH₃, -(CH₂)ₙCH₃, -(CH₂)ₙSi(CH₃)₃, -(CH₂)ₙNH(CH₃)₂, -O(CH₂)ₙCH₃, -O(CH₂)ₙNH₂, -NH(CH₂)ₙNH₂, -(OCH₂CH₂)ₙNH₂, or 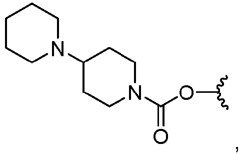 wherein, n represents an integer from 1 to 3; or
R₁ and R₂ are connected to each other to form -(CH₂)ₚ-, -X(CH₂)₍ₚ₋₁₎-, -(CH₂)₍ₚ₋₁₎X-, -X(CH₂)_{(q-1)}X-, or -(CH₂)_{(q-1)}X(CH₂)_{(q-1)}, p represents an integer from 2 to 4, q represents an integer from 2 to 3, and X represents O, NH, or S;
R₃ is -H, -CH₃, -(CH₂)ₙCH₃, -OCH₃, -O(CH₂)ₙCH₃, -NHCH₃, -NH(CH₂)ₙCH₃, -(CH₂)ₙSi(CH₃)₃, -(CH₂)ₙN(CH₃)₂, -(CH₂)ₙCH=CH₂, -NO₂, -F, -Cl, -Br, or -I, and n represents an integer from 1 to 3;
R₄ is -H, -CH₃, -OCH₃, -(CH₂)ₙCH₃, -Si(CH₃)₂C(CH₃)₃, -(CH₂)ₙSi(CH₃)₃, -(CH₂)ₙNHCH(CH₃)₂, -CH₂NH(CH₂)ₙCH₃, -CH₂O(CH₂)ₙCH₃, -F, -Cl, -Br, -I, 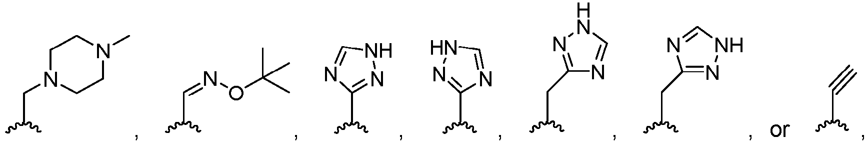 wherein, n represents an integer from 1 to 3; or
R₃ and R₄ are connected to each other to form:

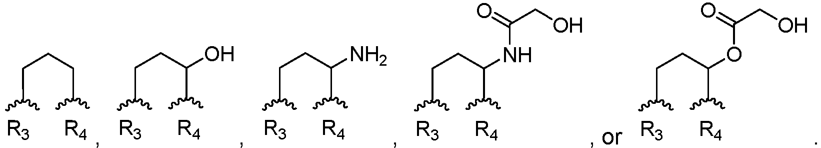

3. The dideuterated camptothecin derivative, the stereoisomer thereof, the pharmaceutically acceptable salt thereof, or the prodrug thereof according to claim 1, 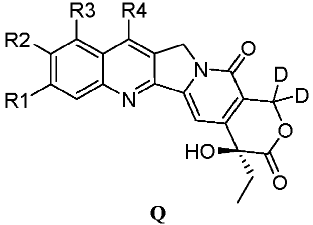 wherein:
R₁ is -H, -F, -Cl, -Br, or -I;
R₂ is -H, -OH, -CH₃, -(CH₂)ₙCH₃, or 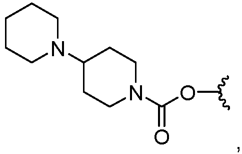 wherein, n represents an integer from 1 to 3; or
R₁ and R₂ are connected to each other to form -X(CH₂)_{(q-1)}X-, q represents an integer from 2 to 3, and X represents O or S;
R₃ is -H, -(CH₂)ₙN(CH₃)₂, -(CH₂)ₙCH=CH₂, or -NO₂, and n represents an integer from 1 to 3;
R₄ is -H, -CH₃, -(CH₂)ₙCH₃, -Si(CH₃)₂C(CH₃)₃, -(CH₂)ₙSi(CH₃)₃, -(CH₂)ₙNHCH(CH₃)₂, 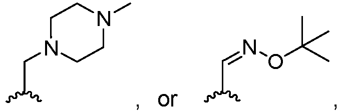 wherein, n represents an integer from 1 to 3; or
R₃ and R₄ are connected to each other to form:

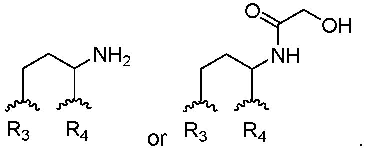

4. The dideuterated camptothecin derivative, the stereoisomer thereof, the pharmaceutically acceptable salt thereof, or the prodrug thereof according to claim 1, 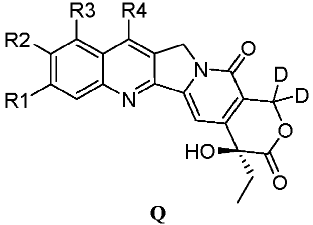 wherein:
R₁ is -H or -F;
R₂ is -H, -OH, -CH₃, or 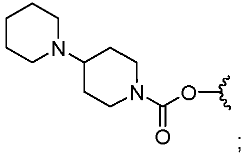 or
R₁ and R₂ are connected to each other to form -O(CH₂)₂O-;
R₃ is -H, -(CH₂)N(CH₃)₂, -CH₂CH=CH₂, or -NO₂;
R₄ is -H, -CH₂CH₃, -Si(CH₃)₂C(CH₃)₃, -(CH₂)₂Si(CH₃)₃, -(CH₂)₂NHCH(CH₃)₂, 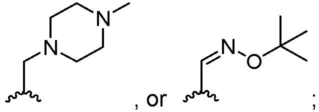 or
R₃ and R₄ are connected to each other to form:

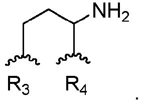

5. The dideuterated camptothecin derivative, the stereoisomer thereof, the pharmaceutically acceptable salt thereof, or the prodrug thereof according to claim 1, 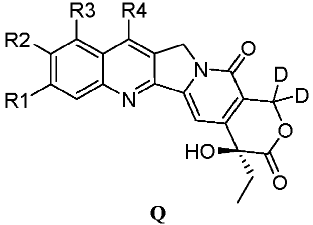 wherein:
R₁ is -H or -F;
R₂ is -H, -OH, or -CH₃;
R₃ is -H;
R₄ is -CH₂CH₃ or -(CH₂)₂NHCH(CH₃)₂; or
R₃ and R₄ are connected to each other to form:

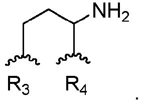

6. The dideuterated camptothecin derivative, the stereoisomer thereof, the pharmaceutically acceptable salt thereof, or the prodrug thereof according to claim 1, wherein the dideuterated camptothecin derivative is selected from the group consisting of:

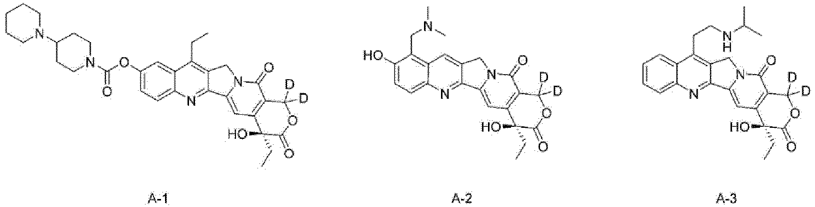

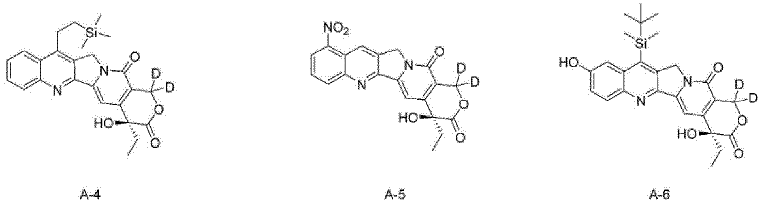

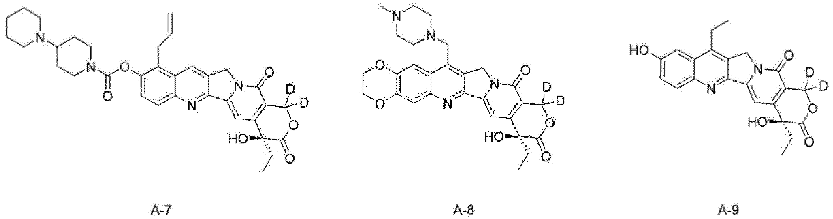

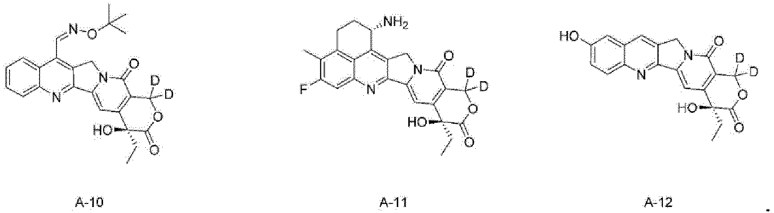

7. A preparation method of the dideuterated camptothecin derivative as shown in general formula Q according to claim 1, wherein the method comprises the following steps: 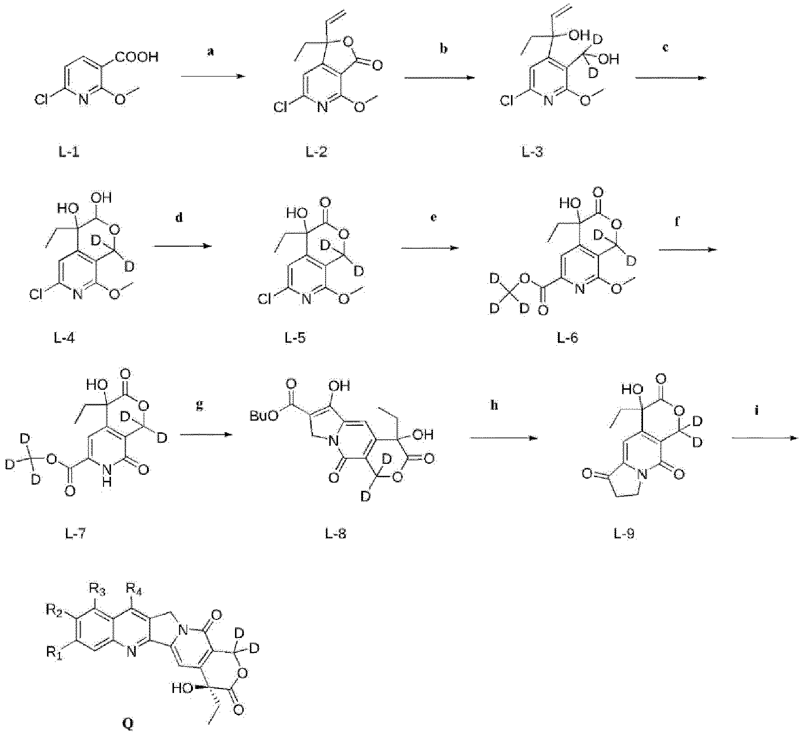
a) reacting compound L-1 with 1-penten-3-one, 2,2,6,6-tetramethylpiperidine, and a lithiation reagent to obtain compound L-2;
b) deuterating compound L-2 with a deuterated reducing agent to obtain compound L-3;
c) subjecting compound L-3 to an ozonization reaction to obtain compound L-4;
d) reacting compound L-4 with 2,2,6,6-tetramethylpiperide oxide, sodium bicarbonate, potassium bromide, and sodium hypochlorite to obtain compound L-5;
e) reacting compound L-5 with a palladium catalyst, a phosphine ligand, a base, and an alcohol in an atmosphere of carbon monoxide to obtain compound L-6;
f) reacting compound L-6 with trimethylchlorosilane and sodium iodide to obtain compound L-7;
g) reacting compound L-7 with a base and an ester to obtain compound L-8;
h) decarboxylating compound L-8 in toluene as a solvent in the presence of trifluoroacetic acid to obtain compound L-9; and
i) reacting compound L-9 with a polysubstituted benzene ring 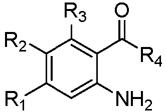 in the presence of a catalyst to obtain compound Q; wherein in the polysubstituted benzene ring, R₁ to R₄ are as defined in claim 1.

8. The preparation method according to claim 7, wherein the method comprises the following steps: 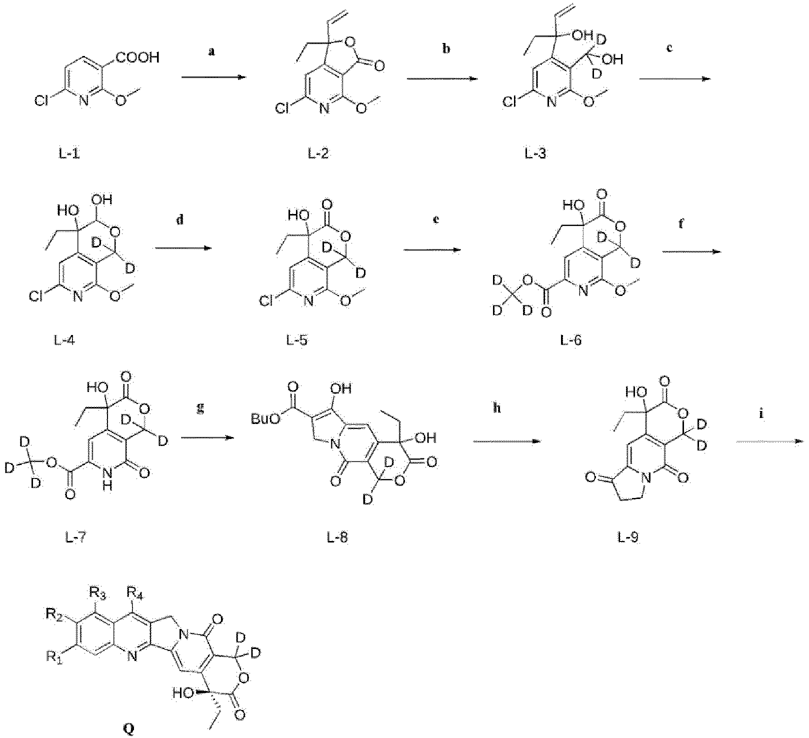
a) reacting compound L-1 with 1-penten-3-one, 2,2,6,6-tetramethylpiperidine, and a lithiation reagent to obtain compound L-2 at a reaction temperature of -78 °C to -10 °C for a reaction time of 2 to 12 hours; wherein the lithiation reagent is selected from the group consisting of n-butyllithium, isobutyllithium, and lithium diisopropylamide; a solvent selected from the group consisting of tetrahydrofuran, methyltetrahydrofuran, ethyl acetate, and petroleum ether is used; and a reaction molar ratio of compound L-1, 1-penten-3-one, 2,2,6,6-tetramethylpiperidine, and the lithiation reagent is 1 : 2 to 4 : 2 to 4 : 3 to 5;
b) deuterating compound L-2 with a deuterated reducing agent to obtain compound L-3; wherein the deuterated reducing agent is selected from the group consisting of deuterated sodium borohydride, deuterated potassium borohydride, deuterated borane, and deuterated lithium aluminum hydride; a solvent selected from the group consisting of dichloromethane, tetrahydrofuran, methanol, and ethanol is used; a reaction molar ratio of compound L-2 to the deuterated reducing agent is 1 : 1.1 to 2.5; a reaction temperature is 0 °C to 40 °C, preferably 10 °C to 35 °C, more preferably 20 °C to 30 °C; and a reaction time is 6 to 15 hours, preferably 8 to 14 hours;
c) subjecting compound L-3 to an ozonization reaction to obtain compound L-4, wherein a solvent is selected from the group consisting of ethanol, methanol, tetrahydrofuran, and dichloromethane; a reaction temperature is -78 °C; and a reaction time is 15 to 30 minutes;
d) reacting compound L-4 with 2,2,6,6-tetramethylpiperidine oxide, sodium bicarbonate, potassium bromide, and sodium hypochlorite to obtain compound L-5; wherein a reaction molar ratio of compound L-4, 2,2,6,6-tetramethylpiperidine oxide, sodium bicarbonate, potassium bromide, and sodium hypochlorite is 1 : 0.01 to 0.1 : 0.1 to 0.5 : 0.1 to 0.5 : 1 to 5; and a reaction temperature is -10 °C to 10 °C;
e) reacting compound L-5 with a palladium catalyst, a phosphine ligand, a base, and an alcohol in the atmosphere of carbon monoxide to obtain compound L-6; wherein the base is selected from the group consisting of potassium carbonate, sodium carbonate, cesium carbonate, potassium phosphate, and triethylamine; the palladium catalyst is selected from the group consisting of palladium acetate, bistriphenylphosphine palladium dichloride, 1,1-bis(diphenylphosphine)ferrocene palladium dichloride, and triphenylphosphine palladium; the alcohol is deuterated methanol or deuterated ethanol; the phosphine ligand is 1,3-bis(diphenylphosphine)propane; a reaction molar ratio of compound L-5, the palladium catalyst, the phosphine ligand, and the base is 1 : 0.01 to 0.1 : 0.01 to 0.1 : 1.5 to 3; a solvent is N,N-dimethylformamide; a reaction temperature is 60 °C to 120 °C; and a reaction time is 6 to 48 hours;
f) reacting compound L-6 with trimethylchlorosilane and sodium iodide to obtain compound L-7; wherein a reaction molar ratio of compound L-6, trimethylchlorosilane, and sodium iodide is 1 : 2 to 3 : 2 to 3; a solvent is selected from the group consisting of acetonitrile, tetrahydrofuran, dichloromethane, and ethyl acetate; a reaction time is 12 to 24 hours; and a reaction temperature is room temperature;
g) reacting compound L-7 with a base and an ester to obtain compound L-8, wherein a reaction molar ratio of compound L-7, the base and the ester is 1 : 2 to 4 : 2 to 10; a reaction time is 12 to 48 hours; a reaction temperature is 50 °C; and compound L-8 is a five-membered ring compound; wherein the base is selected from the group consisting of potassium carbonate, cesium carbonate, sodium carbonate, and sodium ethoxide; the ester is selected from the group consisting of methyl acrylate, ethyl acrylate, tert-butyl acrylate, and n-butyl acrylate; and a solvent is N,N-dimethylformamide or dimethyl sulfoxide;
h) decarboxylating compound L-8 in toluene as a solvent in the presence of trifluoroacetic acid to obtain compound L-9; wherein a reaction temperature is 80 °C to 140 °C, and a reaction time is 2 to 6 hours; and
i) reacting compound L-9 with a polysubstituted benzene ring 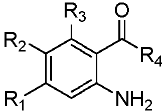 in the presence of a catalyst to obtain compound Q; wherein a reaction molar ratio of compound L-9, the polysubstituted benzene ring, and the catalyst is 1 : 1 : 0.2 to 0.6; a solvent selected from the group consisting of tetrahydrofuran, ethanol, and toluene is used for reaction; the catalyst is selected from anhydrous p-toluene sulfonic acid, pyridinium p-toluenesulfonate, antimony trichloride, sulfuric acid, and concentrated hydrochloric acid; a reaction temperature is 80 °C to 140 °C; a reaction time is 12 to 48 hours; and in the polysubstituted benzene ring, R₁ to R₄ are as defined in claim 1.

9. A preparation method of compound L-3, wherein the method comprises the following step: 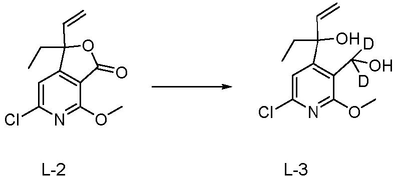
reacting compound L-2 with a deuterated reducing agent in solvent I to obtain compound L-3,
wherein, the deuterated reducing agent is selected from the group consisting of deuterated sodium borohydride, deuterated potassium borohydride, deuterated borane, and deuterated lithium aluminum hydride; the solvent I is selected from the group consisting of dichloromethane, tetrahydrofuran, methanol, and ethanol; a reaction molar ratio of compound L-2 to the deuterated reducing agent is 1 : 1.1 to 2.5; a reaction temperature is 0 °C to 40 °C; and a reaction time is 6 to 15 hours.

10. A preparation method of compound L-6, wherein the method comprises the following step: 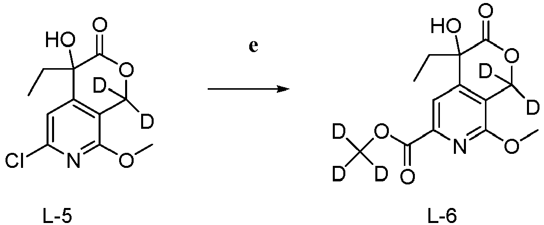
e) reacting compound L-5 with a palladium catalyst, a phosphine ligand, a base, and an alcohol in an atmosphere of carbon monoxide to obtain compound L-6,
wherein, the base is selected from the group consisting of potassium carbonate, sodium carbonate, cesium carbonate, potassium phosphate, and triethylamine; the palladium catalyst is selected from the group consisting of palladium acetate, bistriphenylphosphine palladium dichloride, 1,1-bis(diphenylphosphine)ferrocene palladium dichloride, and triphenylphosphine palladium; the alcohol is deuterated methanol or deuterated ethanol; the phosphine ligand is 1,3-bis(diphenylphosphine)propane; a reaction molar ratio of compound L-5, the palladium catalyst, the phosphine ligand, and the base is 1 : 0.01 to 0.1 : 0.01 to 0.1 : 1.5 to 3; a solvent is N,N-dimethylformamide; a reaction temperature is 60 °C to 120 °C; and a reaction time is 6 to 48 hours.
